# EUROPEAN PATENT SPECIFICATION

(11) **EP 3 377 799 B1**
(45) Date of publication and mention of the grant of the patent: **07.10.2020**
(21) Application number: 16798458.2
(22) Date of filing: 16.11.2016
(51) Int. Cl.: B60B 33/00, B60B 33/08, G01N 33/00, F16L 55/32, B60B 19/00, B62D 55/02, F16L 101/30, B62D 55/265

(54) **PIPELINE INSPECTION ROBOT**
PRÜFROBOTER FÜR PIPELINE
ROBOT D'INSPECTION DE CONDUITE

(30) Priority: 20.11.2015 GB 201520462
(43) Date of publication of application: 26.09.2018
(73) Proprietor: National Grid Gas Plc., London WC2N 5EH (GB)
(72) Inventor: CICHOSZ, Rafal, Harrogate HG2 8BU (GB); WHITE, John, Leeds LS18 4NS (GB); PRICE, Tom, York YO31 0UL (GB); BARKER, Chris, Leeds LS9 0EH (GB)
(74) Representative: Heinonen & Co
(86) International application number: PCT/EP2016/077861
(87) International publication number: WO 2017/085126

(56) References cited:
- WO-A1-2015/081013
- WO-A1-2016/075428
- US-A1- 2012 116 583
- US-A1- 2014 076 223

## Description

### TECHNICAL FIELD

The present invention relates to a mobile robot for internally inspecting pipelines, a robotic system comprising two or more such robots and to a method for pipeline inspection as defined in the present independent claims.

### BACKGROUND OF THE INVENTION

Typical pipe constructions such as sewers, gas/oil transmission pipelines, gas/oil distribution pipes are suffering from several diseases when getting old. (Note: A "pipeline" or "pipe" may alternatively be termed simply as "conduit", and as used herein such terms may be used interchangeably). Aging, corrosion and mechanical stress generally lead to the loss of material thickness or generation of cracks that can cause leakages or sometimes the destruction of the pipeline construction

Thus, periodic inspection of the pipe system is required in order to prevent such damages. Since many of these constructions have not been designed to optimize automatic inspection and repair tasks, inspection and maintenance generate huge costs, especially if disassembling or even excavating is necessary. Inspection and maintenance technology has then become a growing industry and a large variety of systems have been developed.

For the maintenance of pipelines devices known as "pigs" are used often. "Pig" is sometimes claimed as an acronym or backronym derived from the initial letters of the term "pipeline inspection gauge" or "pipeline intervention gadget". Accordingly, "pigging" in the context of pipelines refers to the practice of using "pigs" to perform various maintenance operations on a pipeline. This is done without stopping the flow of the product in the pipeline. These operations include but are not limited to cleaning and inspecting the pipeline. This is accomplished by inserting the pig into a "pig launcher" (or "launching station") - an oversized section in the pipeline, reducing to the normal diameter. The launcher is then closed and the pressure-driven flow of the product in the pipeline is used to push the pig along down the pipe until it reaches the receiving trap - the "pig catcher" (or "receiving station").

Such "pigs" are often designed to be as small and lightweight as possible, and to that end it is common practice to provide such apparatuses or "robots" with a multi-strand or multi-tube tether or umbilical cable. An umbilical cable is a cable that allows the robot to be provided in communication with an external body (such as an above-ground control station). The cable may for example enable the robot to be in electrical communication (allowing data signals to be exchanged and/or electrical power to be supplied), fluid communication (e.g. allowing fluid such as compressed gas, such as compressed air, or liquid such as water, to be provided to the robot) or optical communication (e.g. allowing optical data signals to be provided via a fibre-optic cable). It is named by analogy with an umbilical cord. The term "communication" in this sense refers to a connection. Such pigs or robots may also comprise several different sections or modules, e.g. each being constructed, designed and controlled to perform a given unique operation in the overall pipeline maintenance procedure.

However, some pipelines are "unpiggable", for example because a free swimming tool can't be introduced or removed. Other barriers to pigging are insufficient flow to overcome friction and drive a pig, multi-diameters pipes or internal obstacles.

Some possible alternative locomotion strategies used to solve the in-pipe inspection problems are robots of the wheel type, caterpillar type, wall-pressed type, walking type, inch-worm type or screw type. Bends of the pipe are usually overcome thanks to differential-drive steering (for single body systems) or articulated structures.

When climbing ability is required, the most common solution is to use robots with spreading systems. However, none of these systems can deal with narrow pipe environments which integrate high abrupt diameter changes and bends and also require climbing ability. In this case, it has been tried to combine the locomotion system of the robots with attachment elements such as grasps, suction cups, adhesive polymers or (electro)magnetic elements. Since these concepts usually imply complex mechanics and since the considered environment is ferromagnetic magnetic attachment systems have been considered. These robots take advantage of the magnetic force in order to travel on surfaces with any inclination, however, their mobility is limited to smooth obstacle-free surfaces.

Publications US 2014/076223 A1, WO 2015/081013 A1, and US 2012/116583 disclose examples of prior art robots with limited mobility capabilities in challenging pipe environments.

Accordingly, there is still a substantial need for mobile robots which are suitable to inspect complex shaped pipe structures. Such complex pipe structures may have a wide range of inner diameters and may be composed of horizontal and vertical pipe elements. In addition, internal obstacles such as bends and pipeline fittings (T-branches, Y-branches), and any inclination can be encountered.

It is an object of the present invention to provide a mobile robot for pipeline inspection that is capable to address disadvantages associated with the prior art such as discussed above.

### SUMMARY OF THE INVENTION

The present invention provides a robot as defined in present independent claim 1 which is suitable for travel through a pipeline. The inventive robot comprises at least one tracked drive means and at least one roller means that can swivel about an axis substantially normal to a rolling axis thereof, wherein said at least one tracked drive means and at least one roller means are provided with magnetic means for generating a magnetic adhesion force between the robot and an internal wall of the pipeline.

An embodiment of the present invention provides a robot as defined in present claim 2 which is suitable for travel through a pipeline. The inventive robot comprises a body having a streamlined aerofoil shape form that promotes pressing of the robot to the internal wall of the pipeline.

By means of its magnetic locomotion or traction system the inventive robot of an embodiment of the present invention can travel through pipelines with any inclination regarding the gravity vector and can also easily climb into vertical pipeline segments. The magnetic adhesion force between the robot and the internal wall of the pipeline securely maintains the robot in contact with the surface. Internal obstacles such as bends or pipeline fittings (T-branches, Y-branches) are overcome with the help of the tracked drive means in a similar manner as a caterpillar is driving through impassable terrain. The speed of the tracked drive means and their motion or movement direction can be controlled independently, for example, one track rotates forwards and the other backwards, providing steering capability to go through 45° or 90° bends, T-branches and Y-branches.

In another embodiment the present invention provides robotic systems as defined in present claims 14 and 15. In an embodiment of the inventive robot system said system comprises two robots as defined above, wherein said two robots are connected to each other in such a way that their at least one tracked drive means and at least one roller means are arranged opposite to each other and when said robotic system travels through a pipeline are efficiently pressed to the pipeline wall. This robotic system is therefore of the well-known wall-pressed type.

In an embodiment of the present invention, the inventive robots described above may be provided with a multi-strand or multi-tube tether or umbilical cable via which it is linked to an above-ground control station (for example a computer workstation) and sources of electrical power, operational control signals, supplies of pressurised fluid to onboard pneumatic and/or hydraulic systems, and suchlike. Instead of a tether or umbilical cable the communication with the control station may be wireless, e.g. radio, optical or acoustically.

In another embodiment of the inventive robot system said system comprises two or more (a "plurality") robots as defined above. Said robots may be configured to cooperate with one another in such a way that when traveling through a pipeline they are distributed at different respective circumferential locations around the inner bore of the pipeline and move generally parallel to the axis of the pipeline. Optionally, the members of the robot group may be configured to communicate with one another. Optionally, a system of constant feedback between robots may be implemented that allows constant coordination (e.g. adjustment of direction/path of travel or speed) of individual robots in cooperation with others, as well as a change of the behavior of the whole group. The communication may be by only local communication, which, for example, can be achieved by wireless transmission systems. Such a robot system is more resistant to failure. Whereas one large robot may fail and ruin a mission, a group of robots can continue even if several robots fail.

The plurality of robots as defined above may each be configured to communicate with at least one other robot, optionally by a wireless communications link, optionally via a base station, alternatively or in addition directly with one another. The robots may be configured to cooperate to perform an inspection operation.

The inspection operation may comprise travel of the plurality of robots along the pipeline, each robot inspecting a respective area of the pipeline in a coordinated manner wherein substantially the whole of an interior surface of a predetermined length of pipeline is inspected. In some arrangements, substantially the whole of the interior surface of the predetermined length of pipeline is inspected during the inspection operation. The inspection operation may comprise causing the plurality of robots to pass along the predetermined length of pipeline a predetermined number of times, which may be one, two, three or more times.

Each path followed by a robot over the predetermined length during the inspection operation may be substantially unique. Optionally, no robot follows the same path more than once during a given inspection operation. Further optionally no two robots follow substantially the same path during a given inspection operation.

Each time a robot travels along the predetermined length it may inspect a portion of the pipeline that is not inspected more than once during a given inspection operation.

The plurality of robots may be arranged to inspect the pipeline by means of one or more NDT sensor devices such as by means of acoustic resonance inspection, ultrasonic inspection, eddy current inspection, capture of one or more images such as video images or any other suitable method. The robots may be configured to detect the presence of one or more chemical species in some embodiments.

Each of the plurality of robots may comprise a body having a streamlined aerofoil shape form that promotes pressing of the robot to the internal wall of the pipeline.

The present invention can be applied in a method for pipeline inspection. The method comprises moving at least one robot or robotic system as defined above along a pipeline within a pipeline network; inspecting said pipeline for leaks or failures using the at least one machine vison system and/or the at least one NDT device and/or the at least one other sensor device of said robot or robotic system; and tracking the position of said robot or robotic system within said pipeline using the at least one locating device, e.g. a global positioning system (GPS).

Other aspects, objects and advantages of the invention will be apparent from the following detailed description of exemplary or preferred embodiments considered in conjunction with the accompanying drawings of those exemplary or preferred embodiments.

Advantageous and/or preferred embodiments of the invention are subject matter of the respective sub-claims.

Unless otherwise expressly specified, all of the numerical ranges, amounts, values and percentages, such as those for amounts of materials, elemental contents, times and temperatures, ratios of amounts, and others, in the following portion of the specification and attached claims may be read as if prefaced by the word "about" even though the term "about" may not expressly appear with the value, amount, or range. Accordingly, unless indicated to the contrary, the numerical parameters set forth in the following specification and attached claims are approximations that may vary depending upon the desired properties sought to be obtained by the present invention, At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains error necessarily resulting from the standard deviation found in its underlying respective testing measurements.

When percentages by weight are used herein, the numerical values reported are relative to the total weight.

Also, it should be understood that any numerical range recited herein is intended to include all sub-ranges subsumed therein. Recitation of ranges of values herein are merely intended to serve as a shorthand method of referring individually to each separate value falling within the range, unless otherwise indicated herein, and each separate value is incorporated into the specification as if it were individually recited herein. Furthermore, when numerical ranges are set forth herein, these ranges are inclusive of the recited range end points (i.e. end points may be used). For example, a range of "1 to 10" is intended to include all sub-ranges between (and including) the recited minimum value of 1 and the recited maximum value of 10, that is, having a minimum value equal to or greater than 1 and a maximum value of equal to or less than 10.

In one embodiment of the invention, the magnetic roller means of the inventive robot are selected from the group consisting of castor (or caster) wheels and roller-balls wheels. Both types of wheels may be combined if suitable or desired. In general, a castor wheel is an undriven, single, double or compound wheel that is designed to be mounted to the bottom of a larger object (the "vehicle") so as to enable that object to be easily moved. The castor wheels used in the invention is advantageously a swivel castor. A swivel castor incorporates a wheel mounted to a fork, wherein an additional swivel joint above the fork allows the fork to freely rotate about 360°, thus enabling the wheel to roll in any direction. This makes it possible to easily move the vehicle in any direction without changing it orientation. Roller-ball wheels comprise a roller ball that can freely rotate, for example in a roller-ball socket. The magnets used for the wheels may be permanent magnets or electromagnets. Permanent magnets (for example neodymium magnets) are preferred since they do not need electric current and have a lighter weight. Further, they are fail-safe. For example, the wheels may comprise ferromagnetic rings.

In another embodiment of the invention, the magnetic tracked drive means (or caterpillar tracks) of the inventive robot comprise a suspension system allowing the drive tracks to bend and keep constant traction and required tension. Suitable suspension systems are commercially available or may easily be adapted to the practical needs. The magnetic means of the tracked drive means may be the same as for the roller means. The shape of the tracks and suspension system is adequate to pipe curvature which will increase the contact area and maximise friction. In an embodiment the magnetic tracks are reinforced by metal dowel pins in each joint. The pins are locked in pockets which keep them in place.

In yet another embodiment of the invention, the inventive robot comprises a body having a streamlined aerofoil shape form that promotes pressing of the robot to the internal wall of the pipeline. An aerofoil (or airfoil) is the shape of a wing. An aerofoil-shaped body moved through a fluid produces an aerodynamic force which may be used for the invention's purpose to press the robot down. An example for an aerofoil shape found in nature is the dolphin flipper fin. The streamlined aerofoil shape used in the invention may be passive or active. Passive means that the aerofoil shape does not change in use, whereas active means that the aerofoil shape may change in response to external cues. Active aerofoil shapes may be made of an adaptive material, e.g. a magnetorheological elastomeric (MRE) material. MREs (also called magnetosensitive elastomers) are a class of solids that consist of polymeric matrix with embedded micro- or nano-sized ferromagnetic particles such as carbonyl iron. As a result of this composite microstructure, the mechanical properties of these materials can be controlled by the application of magnetic field. The body of the inventive robot may, for example, have a "monocoque" design or may be comprised of a chassis and a body carried by the chassis. A monocoque is a structural approach whereby loads are supported through an object's external skin, similar to an egg shell. If a chassis is used it do not need to have an aerofoil shape. It some pipeline environments it may be advantageous that the body (or chassis/body) is pressure-resistant and gas-tight.

In another embodiment of the invention, the inventive robot may comprise at least one machine vision (MV) system. MV is the technology and methods used to provide imaging-based automatic inspection and analysis for such applications as automatic inspection, process control, and robot guidance in industry. In the inventive robot the MV system may be mounted, for example, to the front or rear or to the front and rear of the robot. The MV system may comprise at least one video camera, for example, a fixed forwards facing camera, a self-levelling camera or a pan and tilt camera. The camera technology used may be digital or analogue. Analogue cameras are available in three main technologies each of which may be used in the invention: CMOS, CCD and CCIQ. These camera types are commercially available in a large selection. In some pipeline environments it may be advantageous that the camera is pressure-resistant and gas-tight. The video camera may operate in the visible or non-visible light spectrum. The non-visible light spectrum may be, for example, the infra-red spectrum, the near-infrared spectrum or the ultra-violet spectrum. In addition the video camera may comprise at least one lighting array for illumination. The MV system allows the robot to recognise in pipe features and to self-center relative to the pipeline. It can also be used for location, guidance and as a check for tasks completed by the robot.

In another embodiment of the invention, the inventive robot may comprise at least one non-destructive testing (NDT) device. The NDT device is based on a technique suitable for pipelines, for example magnetic flux leakage, acoustic resonance, ultrasonic or eddy current technique. With these techniques data of the entire circumference of the pipe wall can be provided for structural evaluations. Defects such as pit corrosion and general corrosion, cracks, dents, wrinkles and buckles, coating disbondment, wall thickness and metal loss can be detected.

The NDT device may be, for example, mounted to the robot via a manipulator or robotic arm allowing to move the NDT device within the pipeline. In an embodiment of the present invention the movable arm may carry at least one sensor portion, the sensor portion comprising at least one sensor, the robot being configured to move the arm from a lowered position to a raised position with respect to an upright orientation of the robot to allow the at least one sensor to be positioned substantially coincident with a centreline of a substantially cylindrical pipeline, in use.

In some situations, in the raised position the sensor portion may be positioned so that it is "substantially coincident" with the centreline of the pipeline. The amount by which the sensor portion is raised from the lowered position may be adjusted to accommodate use in pipelines of different respective internal diameters. Therefore the height of the arm may be adjusted so that the sensor portion is "substantially coincident" with the centreline.

The arm may be configured to cause the sensor portion to remain in a substantially fixed orientation with respect to the centreline of the pipeline when the sensor portion is raised and lowered. For example, the robot may be configured such that as the sensor portion is raised or lowered, it experiences translation with respect to the centreline of the pipeline with substantially no relative rotation. In some embodiments the arm may comprise a bar linkage arrangement such as a four bar linkage arrangement allowing raising of the sensor portion without rotation thereof.

The sensor portion may comprise at least one non-destructive testing (NDT) device.

Optionally, said at least one NDT device may comprise a magnetic flux detector, optionally in addition a magnetic flux source. Alternatively or in addition the NDT device may comprise an acoustic detector and optionally an acoustic source. The NDT device may be configured to cause acoustic resonance of a portion of a pipe and to measure the frequency of acoustic resonance. Other arrangements may be useful. Alternatively or in addition the NDT device may comprise an ultrasonic receiver and optionally an ultrasonic transmitter. The NDT device may be arranged to perform ultrasonic inspection. Alternatively or in addition the NDT device may be configured to induce and detect electrical eddy currents in the pipeline. Other arrangements may be useful in some embodiments.

There is at least one sensor on the sensor portion that may comprise a device selected from the group consisting of a temperature sensor, a pressure sensor, a flow sensor and a sensor for sensing the presence of one or more chemical substances. The sensor for sensing chemical substances may be one selected from the group consisting of a methane sensor and a Fourier transform IR (FTIR) spectroscope.

In an embodiment of the invention, the NDT device/manipulator arm is, for example, mounted on top of the robot's body, and the manipulator arm allows moving the NDT device to the center of the pipeline. Of course, the NDT devise may also be mounted underneath or to a side of the robot's body if suitable or desired, and may also be mounted without a manipulator arm.

In yet another embodiment of the invention, the inventive robot may comprise at least one sensor device, for example a temperature sensor, a pressure sensor, a flow sensor or sensors for chemical substances. The sensor for chemical substances may be, for example, a methane sensor for detecting methane or, for more advanced chemical analysis, a Fourier transform IR (FTIR) spectroscope. Of course, also other sensors may be used according to the practical circumstances.

In another embodiment of the invention, the inventive robot may comprise at least one locating device for detecting the position of the robot within the pipeline. The locating device may be, for example, an odometer, a gyro/orientation sensor, an inertial sensor (gyro sensors and accelerometers together) and a global positioning system (GPS) or sonde.

Of course, all optional features of the inventive robot as described above may be combined. Thus, an inventive robot may comprise, for example, a front and rear MV system, a NDT device and any additional sensors needed for specific purposes.

Further, the skilled artisan will appreciate that the inventive robot and/or any MV, NDT or sensor devices may have a modular design. Modular design, or "modularity in design", is a design approach that subdivides a system into smaller parts called modules, that can be independently created and then used in different systems. A modular system can be characterized by functional partitioning into discrete scalable, reusable modules, rigorous use of well-defined modular interfaces, and making use of industry standards for interfaces. Besides reduction in cost (due to less customization, and shorter learning time), and flexibility in design, modularity offers other benefits such as augmentation (adding new solution by merely plugging in a new module), and exclusion.

If suitable or desired it is also possible to combine two or more inventive robots in a way that one (or more) robots tow or push the others. Of course, the combined robots may be identical or not. Thus, it is possible that, for example, the MV system, the NDT device and any additional sensors are mounted on different robots.

### BRIEF DESCRIPTION OF THE DRAWINGS

One or more embodiments of the invention in its various aspects will now be described, by way of example only, with reference to the accompanying drawings, in which:
FIGURE 1 is a plan view schematic illustration of a pipeline inspection robot according to an embodiment of the present invention;
FIGURE 2 is a perspective view of the same pipeline inspection robot schematically illustrated in Figure 1;

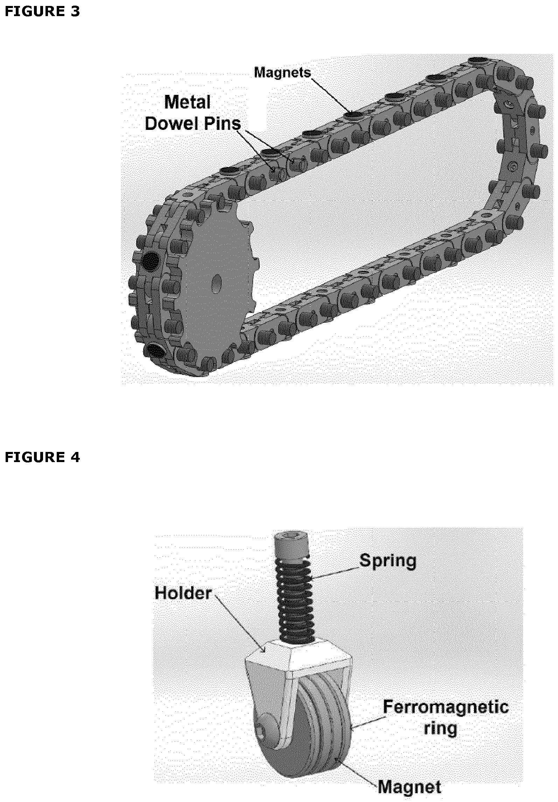

FIGURE 3 is a perspective view of a magnetic tracked drive means according to an embodiment of the present invention;
FIGURE 4 is a perspective view of a magnetic roller means according to an embodiment of the present invention comprising

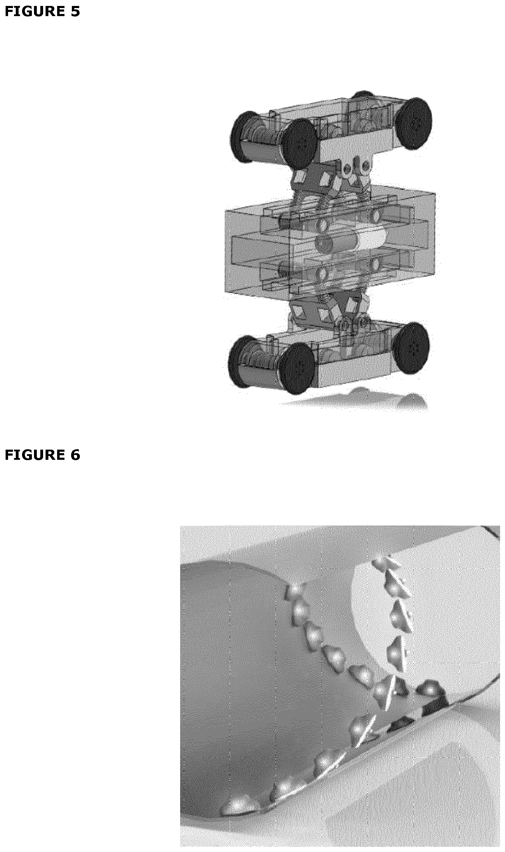

FIGURE 5 is a perspective view of a robotic system according to an embodiment of the invention comprising two robots as defined above, wherein said two robots are connected to each other (here e.g. centre-aligned) in such a way that their at least one tracked drive means and at least one roller means are arranged opposite to each other and when said robotic system travels through a pipeline are efficiently pressed to the pipeline wall.
FIGURE 6 is a perspective view showing an inventive robotic system according to another embodiment of the invention comprising a plurality of robots as defined above. The robots are configured to cooperate with one another in such a way that when traveling through a pipeline they are distributed at different respective circumferential locations around the inner bore of the pipeline and move generally parallel to the axis of the pipeline.

### DETAILED DESCRIPTION OF EMBODIMENTS

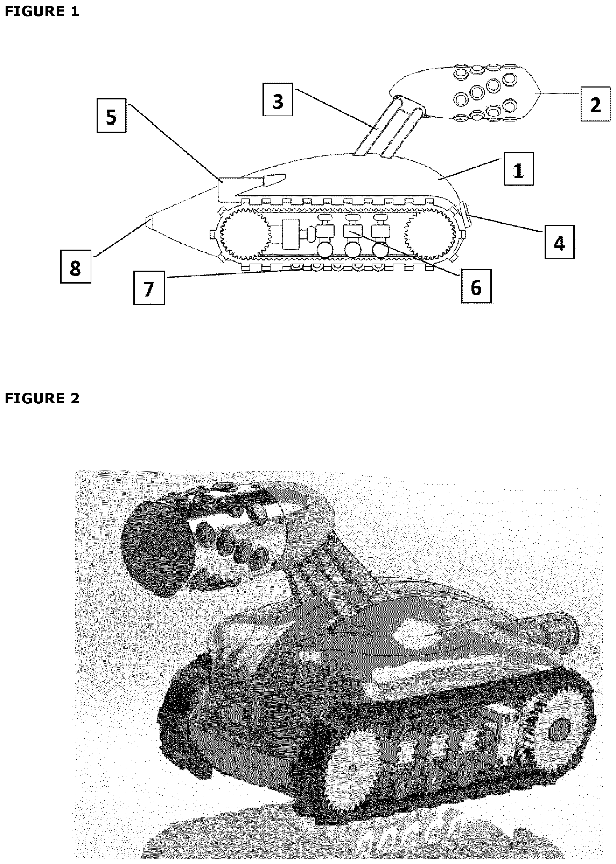

An inventive robot according to one embodiment of the invention is schematically illustrated in Figure 1. It includes a streamlined aerofoil shaped (low profile) body 1 with NDT articulation arm 3 mounted on a top. By means of the articulation system 3 the modular NDT scanning module 2 can be risen to the centre of pipe (in range of DN600 to DN900). The device drive while monitoring the inside of the pipe by forward-looking field camera 4. During normal operation and reversing the umbilical cable used for data and control signal transmission is observed by rear facing camera 5. Further, the shape of the tracks is adapted to complex pipe geometry and to increase the contact surface area. The suspension system 6 allows the tracks to bend and keep constant traction and required tension. A series of magnetic free rolling wheels 7 increase traction and allows the robot to navigate vertical and incline segment of the pipe. The data and control signals are transmitted through an umbilical cable connected to the robot by a rear connector 8.

In an embodiment of the present invention an umbilical management system (UMS) is used. The UMS may be located inside the launch vessel. Suitable UMSs are commercially available and/or may be easily adapted. For example, a suitable UMS has the following features:
- Drum rotation powered by electric motors
- Drum rotation manual override for failure mode recovery
- Wheeled platform to allow smooth insertion of the UMS into the launch vessel
- Motorised umbilical feed to allow the umbilical to be spooled evenly across the drum.
- Integrated high-pressure camera (as defined above) to monitor UMS mechanisms and umbilical spooling

## Claims

1. A robot suitable for travel through a pipeline comprising:
at least one tracked drive means and at least one roller means (7) that can swivel about an axis substantially normal to a rolling axis thereof, wherein said at least one tracked drive means and at least one roller means (7) are provided with magnetic means for generating a magnetic adhesion force between the robot and an internal wall of the pipeline.

2. The robot of claim 1, wherein said robot comprises a body (1) having a streamlined aerofoil shape form that promotes pressing of the robot to the internal wall of the pipeline.

3. The robot of claim 1 or 2, wherein said roller means (7) is selected from the group consisting of castor wheels and roller-balls wheels.

4. The robot according to anyone of claims 1, 2 and 3 wherein said magnetic means comprises permanent magnets.

5. The robot according to anyone of claims 1, 2, 3 and 4, wherein said tracked drive means comprises a suspension system (6) allowing the drive tracks to bend and keep constant traction and required tension.

6. The robot according to claim 2, wherein said body (1) is made of an adaptive material which responds to external cues.

7. The robot of anyone of the preceding claims further comprising at least one machine vision system (4, 5), wherein said at least one machine vision system is preferably mounted to the front or rear or to the front and rear of the robot.

8. The robot according to claim 7, wherein said machine vision system comprises at least one video camera (4, 5) selected from the group consisting of a fixed forwards facing camera (4), a self-levelling camera and a pan and tilt camera, and wherein said at least one video camera preferably operates in the visible or non-visible light spectrum,.wherein said non-visible light spectrum is preferably selected from the group consisting of the infra-red spectrum, the near-infrared spectrum and the ultra-violet spectrum.

9. The robot according to claim 8, wherein said at least one video camera (4, 5) comprises at least one lighting array.

10. The robot according to anyone of the preceding claims further comprising at least one non-destructive testing (NDT) device (2), wherein said NDT device is preferably based on a technique selected from the group consisting of magnetic flux leakage, acoustic resonance, ultrasonic and eddy current.

11. The robot according to claim 10, wherein said NDT device is mounted to said robot via a manipulator arm (3) allowing to move said NDT device within the pipeline.

12. The robot according to anyone of the preceding claims further comprising at least one sensor device selected from the group consisting of a temperature sensor, a pressure sensor, a flow sensor and sensors for chemical substances, wherein said sensor for chemical substances is preferably selected from the group consisting of a methane sensor and a Fourier transform IR spectroscope.

13. The robot according to anyone of the preceding claims further comprising at least one locating device for detecting the position of the robot within the pipeline, wherein said at least one locating device is preferably selected from the group consisting of an odometer, a gyro/orientation sensor and a global positioning system (GPS).

14. A robotic system comprising two robots as defined in anyone of claims 1 to 13, wherein said two robots are connected to each other in such a way that their at least one tracked drive means and at least one roller means are arranged opposite to each other and when said robotic system travels through a pipeline are efficiently pressed to the pipeline wall.

15. A robotic system comprising two or more robots as defined in anyone of claims 1 to 13, wherein said robots are configured to cooperate with one another in such a way that when traveling through a pipeline they are distributed at different respective circumferential locations around the inner bore of the pipeline and move generally parallel to the axis of the pipeline.

## Patentansprüche

1. Roboter, der zum Durchqueren einer Pipeline geeignet ist, umfassend:
mindestens ein Kettenantriebsmittel und mindestens ein Rollmittel (7), das sich um eine Achse, die im Wesentlichen normal zu einer Rollachse davon verläuft, drehen kann, wobei das mindestens eine Kettenantriebsmittel und das mindestens eine Rollmittel (7) mit magnetischen Mitteln versehen sind, um zwischen dem Roboter und einer Innenwand der Pipeline eine magnetische Haftkraft zu erzeugen.

2. Roboter nach Anspruch 1, wobei der Roboter einen Körper (1) umfasst, der eine stromlinienförmige Tragflügelform aufweist, die das Drücken des Roboters an die Innenwand der Pipeline unterstützt.

3. Roboter nach Anspruch 1 oder 2, wobei das Rollmittel (7) aus der Gruppe bestehend aus Lenkrollen und Rollkugeln ausgewählt ist.

4. Roboter nach einem der Ansprüche 1, 2 und 3, wobei die magnetischen Mittel Permanentmagnete umfassen.

5. Roboter nach einem der Ansprüche 1, 2, 3 und 4, wobei das Kettenantriebsmittel ein Aufhängungssystem (6) umfasst, das den Triebschienen gestattet, sich zu biegen und eine konstante Zugkraft und gewünschte Spannung zu halten.

6. Roboter nach Anspruch 2, wobei der Körper (1) aus einem adaptiven Material hergestellt ist, das auf externe Signale anspricht.

7. Roboter nach einem der vorhergehenden Ansprüche, ferner umfassend mindestens ein Bildverarbeitungssystem (4, 5), wobei das mindestens eine Bildverarbeitungssystem vorzugsweise an die Front- oder Rückseite oder an die Front- und Rückseite des Roboters montiert ist.

8. Roboter nach Anspruch 7, wobei das Bildverarbeitungssystem mindestens eine Videokamera (4, 5) umfasst, die aus der Gruppe bestehend aus einer festen, vorwärts gerichteten Kamera (4), einer selbstnivellierenden Kamera und einer Schwenk-Neige-Kamera ausgewählt ist, und wobei die mindestens eine Videokamera vorzugsweise im sichtbaren oder nichtsichtbaren Lichtspektrum arbeitet, wobei das nichtsichtbare Lichtspektrum vorzugsweise aus der Gruppe bestehend aus dem Infrarotspektrum, dem Nah-Infrarotspektrum und dem Ultraviolettspektrum ausgewählt ist.

9. Roboter nach Anspruch 8, wobei die mindestens eine Videokamera (4, 5) mindestens ein Beleuchtungsarray umfasst.

10. Roboter nach einem der vorhergehenden Ansprüche, ferner umfassend mindestens eine Zerstörungsfreie-Prüfungs(NDT)-Vorrichtung (2), wobei die NDT-Vorrichtung vorzugsweise auf einer Technik basiert, ausgewählt aus der Gruppe bestehend aus magnetischer Streuflussprüfung, akustischer Resonanz, Ultraschall und Wirbelstrom-Methode.

11. Roboter nach Anspruch 10, wobei die NDT-Vorrichtung über einen Manipulatorarm (3), der der NDT-Vorrichtung gestattet, sich innerhalb der Pipeline zu bewegen, an dem Roboter montiert ist.

12. Roboter nach einem der vorhergehenden Ansprüche, ferner umfassend mindestens eine Sensorvorrichtung, die aus der Gruppe bestehend aus einem Temperatursensor, einem Drucksensor, einem Flusssensor und Sensoren für chemische Substanzen ausgewählt ist, wobei der Sensor für chemische Substanzen vorzugsweise aus der Gruppe bestehend aus einem Methansensor und einem Fourier-Transformations-Infrarotspektrometer ausgewählt ist.

13. Roboter nach einem der vorhergehenden Ansprüche, ferner umfassend mindestens eine Ortungsvorrichtung zum Detektieren der Position des Roboters innerhalb der Pipeline, wobei die mindestens eine Ortungsvorrichtung vorzugsweise aus der Gruppe bestehend aus einem Wegmesser, einem Gyro-/Orientierungssensor und einem Satellitennavigationssystem (GPS) ausgewählt ist.

14. Robotersystem, umfassend zwei Roboter wie in einem beliebigen der Ansprüche 1 bis 13 definiert, wobei die zwei Roboter derart miteinander verbunden sind, dass ihre mindestens einen Kettenantriebsmittel und mindestens einen Rollmittel gegenüberstehend angeordnet sind und effizient an die Pipelinewand gedrückt werden, wenn das Robotersystem eine Pipeline durchquert.

15. Robotersystem, umfassend zwei oder mehr Roboter wie in einem beliebigen der Ansprüche 1 bis 13 definiert, wobei die Roboter ausgelegt sind, um derart miteinander zusammenzuarbeiten, dass sie beim Durchqueren einer Pipeline an verschiedenen jeweiligen Umfangspositionen um die Innenbohrung der Pipeline verteilt sind und sich im Allgemeinen parallel zur Achse der Pipeline bewegen.

## Revendications

1. Robot approprié pour se déplacer à travers un pipeline, comprenant :
au moins un moyen d'entraînement à chenille et au moins un moyen formant rouleau (7) qui peut pivoter autour d'un axe sensiblement perpendiculaire à un axe de roulement de celui-ci, dans lequel l'au moins un moyen d'entraînement à chenille et l'au moins un moyen formant rouleau (7) sont dotés de moyens magnétiques pour générer une force d'adhérence magnétique entre le robot et une paroi interne du pipeline.

2. Robot selon la revendication 1, dans lequel ledit robot comprend un corps (1) ayant une forme de profil aérodynamique simplifié qui favorise la pression du robot contre la paroi interne du pipeline.

3. Robot selon la revendication 1 ou 2, dans lequel ledit moyen formant rouleau (7) est sélectionné dans le groupe formé par des roues pivotantes et des roues à roulements à billes.

4. Robot selon l'une quelconque des revendications 1, 2 et 3, dans lequel lesdits moyens magnétiques comprennent des aimants permanents.

5. Robot selon l'une quelconque des revendications 1, 2, 3 et 4, dans lequel ledit moyen d'entraînement à chenille comprend un système de suspension (6) permettant aux chenilles d'entraînement de se plier et conserver une traction constante et une tension requise.

6. Robot selon la revendication 2, dans lequel ledit corps (1) est constitué d'un matériau adaptif qui répond à des repères externes.

7. Robot selon l'une quelconque des revendications précédentes, comprenant en outre au moins un système de vision artificielle (4, 5), dans lequel ledit au moins un système de vision artificielle est de préférence monté sur l'avant ou l'arrière ou l'avant et l'arrière du robot.

8. Robot selon la revendication 7, dans lequel ledit système de vision artificielle comprend au moins une caméra vidéo (4,5) sélectionnée dans le groupe formé par une caméra fixe orientée vers l'avant (4), une caméra de mise à niveau automatique et une caméra panoramique et inclinable, et dans lequel ladite au moins une caméra vidéo fonctionne de préférence dans le spectre de lumière visible et non-visible, dans lequel ledit spectre de lumière non-visible est de préférence sélectionné dans le groupe formé par le spectre infrarouge, le spectre infrarouge proche, et le spectre ultraviolet.

9. Robot selon la revendication 8, dans lequel ladite au moins une caméra vidéo (4, 5) comprend au moins un système d'éclairage.

10. Robot selon l'une quelconque des revendications précédentes, comprenant en outre au moins un dispositif d'essai non destructif (NDT) (2), dans lequel ledit dispositif NDT est de préférence basé sur une technique sélectionnée dans le groupe formé par une fuite de flux magnétique, une résonance acoustique, des ultrasons et courants de Foucault.

11. Robot selon la revendication 10, dans lequel ledit dispositif NDT est monté sur ledit robot via un bras de manipulation (3) permettant de faire bouger ledit dispositif NDT dans le pipeline.

12. Robot selon l'une quelconque des revendications précédentes, comprenant en outre au moins un dispositif de détection sélectionné dans le groupe formé par un capteur de température, un capteur de pression, un débitmètre, et des détecteurs pour des substances chimiques, dans lequel ledit détecteur pour des substances chimiques est de préférence sélectionné dans le groupe formé par un détecteur de méthane et un spectroscope IR à transformée de Fourrier.

13. Robot selon l'une quelconque des revendications précédentes, comprenant au moins un dispositif de localisation pour détecter la position du robot dans le pipeline, dans lequel ledit au moins un dispositif de localisation est de préférence sélectionné dans le groupe formé par un odomètre, un capteur gyroscopique/d'orientation, et un système de positionnement global (GPS).

14. Système robotique comprenant deux robots tels que définis dans l'une quelconque des revendications 1 à 13, dans lequel lesdits deux robots sont raccordés l'un à l'autre de manière à ce que leur au moins un moyen d'entraînement à chenille et au moins un moyen formant rouleau soient disposés en opposition l'un par rapport à l'autre et, lorsque ledit système robotique se déplace à travers un pipeline, soient pressés efficacement contre la paroi de pipeline.

15. Système robotique comprenant deux ou davantage de robots tels que définis dans l'une quelconque des revendications 1 à 13, dans lequel lesdits robots sont configurés pour coopérer les uns avec les autres de manière à ce que, lors du déplacement à travers le pipeline, ils soient répartis à différents endroits circonférentiels respectifs autour de l'alésage intérieur du pipeline et se déplacent globalement en parallèle à l'axe du pipeline.
